# EUROPÄISCHE PATENTANMELDUNG

(11) **EP 0 834 547 A2**
(43) Veröffentlichungstag der Anmeldung: **08.04.1998**
(21) Anmeldenummer: 97116516.2
(22) Anmeldetag: 23.09.1997
(51) Int. Cl.: C11D 1/12, C07C 303/22

(54) **Tensidmischungen mit einem Gehalt an Acyloxialkansulfonaten**

(30) Priorität: 01.10.1996 DE 19640573
(71) Anmelder: Clariant GmbH, 65929 Frankfurt am Main (DE)
(72) Erfinder: Nestler, Bernd, Dr., 65929 Frankfurt (DE)

(57) **Zusammenfassung**

Tensidmischungen mit einem Gehalt an Acyloxialkansulfonaten, die durch Mischen verschiedener Acyloxialkansulfonate hergestellt werden, wobei die Gesamtmenge an Acyloxialkansulfonaten ein Gemisch aus Ammonium- und Alkali- und/oder Erdalkalikationen enthält.

## Beschreibung

Acyloxialkansulfonate stellen anionische Tenside dar, die als Rohstoffe für Syndet-Seifen, kosmetische Mittel und Reinigungsformulierungen Verwendung finden. Sie zeichnen sich durch gute Schaumeigenschaften, gute Hartwasserstabilität und gute Hautverträglichkeit aus.

Nachteilig für die Verwendung dieser Tenside ist, daß es sich dabei meist um spröde Feststoffe handelt, die erst bei hohen Temperaturen schmelzen bzw. rührbar sind. Bei diesen hohen Temperaturen, die erforderlich sind, um das Acyloxialkansulfonat überhaupt erst verarbeitbar machen, ist dieses sehr oxidationsempfindlich, eine thermische Zersetzung setzt ein und Verfärbungen treten auf.

Demnach ist es vorteilhaft den Schmelzpunkt bzw. die Temperatur, bei der Acyloxialkansulfonate rührbar und somit verarbeitbar sind, zu senken. Zur Lösung dieses Problems ist es bereits bekannt, solche Acyloxialkansulfonate herzustellen, die gemischte Salze darstellen, wo also das Kation aus einem Gemisch von zwei unterschiedlichen Kationen, beispielsweise Natrium- und Kalium-Ion besteht (US 3 029 264). Diese Acyloxialkansulfonate, wobei es sich vorzugsweise um Acylisethionate handelt, werden durch Veresterung von Fettsäuren mit Mischungen von Salzen der Isethionsäure hergestellt, wobei sich diese Salze in ihren Kationen unterscheiden. Auf diese Weise läßt sich die Bildung von Verfärbungen vermeiden.

Acyloxialkansulfonaten mit gemischten Kationen sind auch in WO 94/09107 beschrieben. Als Kationen kommen dort ausschließlich Magnesium, Kalium und Natrium in Frage.

Gegenstand der Erfindung sind Tensidmischungen mit einem Gehalt an Acyloxialkansulfonaten, hergestellt durch Umsetzung einer oder mehrerer Fettsäuren mit mindestens einem Ammonium-, Alkali- und/oder Erdalkali-hydroxyalkansulfonat unter Bildung eines ersten Acyloxialkansulfonats, wobei vor, während oder nach der Herstellung dieses ersten Acyloxialkansulfonats mindestens ein zweites Ammonium-, Alkali- und/oder Erdalkali-acyloxialkansulfonat zugesetzt wird, wobei die Kationen in dem Hydroxyalkansulfonat und in dem zweiten Acyloxialkansulfonat so gewählt sind, daß die Gesamtmenge an Acyloxialkansulfonat in der Tensidmischung als gemischtes Ammonium- sowie Alkali- und/oder Erdalkali-Salz vorliegt.

Bevorzugt sind erfindungsgemäße Tensidmischungen, die so hergestellt werden, daß darin die Acyloxialkansulfonate mit folgenden molaren Verhältnissen der Kationen vorliegen:
K:NH₄ von 97:3 bis 9:95; Na:NH₄ von 98:2 bis 5:95, insbesondere 97:3 bis 50:50; Na:NH(C₂H₅)₃ von 98:2 bis 10:90; K:NH(C₂H₅)₃ von 98:2 bis 10:90.

Als Fettsäuren kommen gesättigte oder ungesättigte Fettsäuren in Frage mit einem Gehalt von 8 bis 32 C-Atomen. Als Beispiele seien genannt Capronsäure, Caprinsäure, Laurinsäure, Myristinsäure, Stearinsäure, Arachinsäure, Ölsäure, Linolsäure, Linolensäure. Bevorzugt sind Mischungen von Fettsäuren, wie beispielsweise Cocosfettsäure und Talgfettsäure. Neben unverzweigten sind auch verzweigte Fettsäuren geeignet, beispielsweise 2-Ethylhexansäure, 2-Pentyloctansäure, 2-Butylnonansäure, 2-Propyldecansäure, 2-Ethylundecansäure, 2-Butylundecansäure, 2-Methyldodecansäure, 2-Ethyltridecansäure und 2-Methyltetradecansäure und Mischungen davon.

Die Hydroxyalkansulfonate entsprechen der Formel HO-R¹-SO₃X, worin R¹ -CH₂CH₂-, -(CH₂)₃-, -CH₂CH(CH₃)- oder -CH₂CH₂OCH₂CH₂- ist, wobei Ethylen bevorzugt ist.
X bedeutet hierin entweder ein Ammoniumion, vorzugsweise ein Ammoniumion der Formel R¹, R², R³, R⁴N^{⊕}, worin R¹, R¹, R³ und R⁴ gleich oder verschieden sein können und Wasserstoff, C₁-C₄-Alkyl oder C₁-C₄-Hydroxyalkyl, oder ein Alkali- und/oder Erdalkalikation, bedeuten. Bevorzugt sind Natrium; Kalium, ⊕NH(CH₃)₃; ⊕NH(C₂H₅)₃; ⊕NH(CH₂CH₂OH)₃ und ⊕NH₄, X kann auch ein Gemisch verschiedener Ammoniumionen bedeuten.

Die Umsetzung der Fettsäure mit dem Hydroxyalkansulfonat erfolgt nach an sich bekannten Verfahren, vorzugsweise nach dem Verfahren der sogenannten Direktveresterung durch Reaktion eines Überschusses von Fettsäure mit dem Hydroxialkansulfonat in Gegenwart eines Veresterungskatalysators bei einer Temperatur von 100 bis 260°C unter gleichzeitiger Entfernung von vorhandenem Wasser. Diese Direktveresterung erfolgt im einzelnen analog zu den Angaben in EP-A-0 585 071 (US 5,384,421), die hier miteinbezogen wird.

Die Hydroxyalkansulfonsäuresalze können als solche eingesetzt werden, bevorzugt werden sie in Form einer wäßrigen Lösung, im allgemeinen als 40 bis 65 gew.-%ige Lösung, eingesetzt.

Geeignete Veresterungskatalysatoren sind in der genannten EP-A-0 585 071 ausführlich beschrieben. Es handelt sich um Alkansulfonsäuren, Hydroxyalkansulfonsäuren, Arylsulfonsäuren, anorganische Säuren wie Schwefelsäure, Phosphorsaure, phosphorige Säure, Borsäure oder deren Anhydride, Schwermetallsalze wie Zinksulfat, Zirkoniumsulfat, Zinkisethionat, Zinkborat, Aluminiumsulfat, Titansulfat oder Wolframphosphat, Metalloxide wie Zinkoxid, Aluminiumoxid, Magnesiumoxid, Ceroxid, Zirkoniumoxid oder Lanthanoxid, ferner um Mischungen von zwei oder mehreren der genannten Katalysatoren und um Seifen, die aus Schwermetallen und Metalloxiden gebildet werden. Ein besonders bevorzugter Veresterungskatalysator ist Zinkoxid. Der Veresterungskatalysator wird in einer Menge von im allgemeinen 0,05 bis 2 Gew.-%, vorzugsweise 0,05 bis 1 Gew.-%, eingesetzt, bezogen auf Hydroxyalkansulfonat.

Die Veresterung kann im einzelnen in der Weise durchgeführt werden, daß man bei Atmosphärendruck die Fettsäure, das oder die Hydroxyalkansulfonate und den Veresterungskatalysator in einem Reaktionsgefäß vorlegt und die Mischung unter Rühren auf die oben angegebene Temperatur erhitzt. Das gegebenenfalls mit den Ausgangskomponenten in die Reaktionsmischung eingebrachte Wasser und das durch die Veresterungsreaktion entstehende Wasser wird dabei kontinuierlich aus der Reaktionsmischung ausgetragen. Darüberhinaus kann es auch sinnvoll sein, im Verlauf der Veresterungsreaktion oder im Anschluß daran einen Teil der überschüssigen Fettsäure abzudestillieren.

Man kann die Veresterungsreaktion auch anfangs bei Atmosphärendruck und dann unter Anwendung eines Vakuums zur schnelleren Austragung des Wassers durchführen. Die Zeit bis zum angestrebten Umsatz von Fettsäure und Hydroxyalkansulfonat liegt bei etwa 4 bis 8 Stunden. Im allgemeinen wird man, zum Beispiel aus Zeitgründen, einen 100%igen Umsatz nicht anstreben, sondern bei einem niedrigeren Prozentsatz, zum Beispiel bei 75 bis 90 Gew.-% Acyloxyalkansulfonat, die Veresterungsreaktion abbrechen, zum Beispiel durch Abkühlen. Das erhaltene Reaktionsprodukt ist bei Raumtemperatur flüssig oder fest.

Um die Viskosität des Reaktionsgemisches zu vermindern, kann man dem Reaktionsgemisch in allen Fällen vor oder während des Abkühlvorgangs sogenannte Konsistenzregler zugeben. Hierfür kommen in Frage beispielsweise Paraffine, wie in EP-A-0 585 071 beschrieben, Fettsäuren, Fettsäureester mit niederen Alkoholen oder Polyethylenglykole oder Mischungen von Konsistenzreglern. Bevorzugt sind freie Fettsäuren, und zwar solche, die eine andere Kettenlänge haben als die zur Herstellung des Acyloxialkansulfonats benutzte Fettsäure. Der Anteil dieser Konsistenzregler kann bis zu 60 Gew.-%, vorzugsweise bis zu 30 Gew.-% betragen. Bevorzugt sind Mischungen mit bis zu 30 Gew.-% Paraffin, bis zu 50 Gew.-% Fettsäure und bis zu 10 Gew.-% Polyethylenglykol. Die Prozentangaben beziehen sich jeweils auf die Summe aller Komponenten.

Es ist von wesentlicher Bedeutung für die erfindungsgemäßen Tensidmischungen, daß die darin enthaltenen Acyloxialkansulfonate in ihrer Gesamtheit nicht ein einziges Kation, sondern ein Gemisch verschiedener Kationen enthalten, wobei mindestens eines dieser Kationen ein Ammoniumkation ist wie oben definiert und der restliche Anteil Alkali- und/oder Erdalkalikationen bzw. weitere Ammoniumkationen ausmacht. Dies wird dadurch erreicht, indem man vor, während oder nach der Herstellung des ersten Acyloxialkansulfonats ein anderes Acyloxialkansulfonat, das hier als zweites Acyloxialkansulfonat bezeichnet weden soll, zumischt.

Dieses zweite Acyloxialkansulfonat kann ebenfalls als Ammoniumsalz, als Alkali- und/oder Erdalkalisalz oder als Salz mit einem Gemisch dieser Kationen vorliegen. Die Art des Kations bei diesem zweiten Acyloxialkansulfonat hängt ab von der Art des Kations in dem ersten Acyloxialkansulfonat, denn die fertige Tensidmischung muß sowohl Ammonium- als auch Alkali- und/oder Erdalkalikationen enthalten. Dies wird beispielsweise dadurch erreicht, daß man als erstes Acyloxialkansulfonat ein Alkali- und/oder Erdalkali-acyloxialkansulfonat herstellt und als zweites Acyloxialkansulfonat ein Ammoniumacyloxialkansulfonat allein oder ein Gemisch aus Ammoniumacyloxialkansulfonat und Alkali- und/oder Erdalkali-acyloxialkansulfonat zumischt. Umgekehrt kann man ausgehend von einem Ammonium-hydroxialkansulfonat ein Ammonium-acyloxialkansulfonat als erstes Acyloxialkansulfonat herstellen und als zweites Acyloxialkansulfonat ein Alkali- oder Erdalkali-acyloxialkansulfonat allein oder ein Gemisch aus Alkali- und/oder Erdalkali-acyloxyalkansulfonat und Ammonium-acyloxialkansulfonat zumischen. Möglich ist auch als erstes Acyloxialkansulfonat ein Gemisch aus Ammonium-acyloxialkansulfonat und Alkali- und/oder Erdalkaliacyloxialkansulfonat herzustellen und als zweites Acyloxialkansulfonat ein Ammonium-, Alkali- und/oder Erdalkali-acyloxialkansulfonat oder ein Gemisch aus Ammonium- und Alkali- und/oder Erdalkali-acyloxialkansulfonat in die erfindungsgemäße Tensidmischung einzubringen. Erfindungsgemäß sind auch solche Tensidmischungen, die drei oder mehr verschiedene Arten von Kationen enthalten, wobei mindestens eines dieser Kationen ein Ammoniumkation sein muß. Beispiele hierfür sind Acyloxialkansulfonate mit einem Gemisch aus Natrium-, Kalium- und NH₄^{⊕}-Ionen.

Das fertige, zweite Acyloxialkansulfonat wird separat hergestellt, beispielsweise nach dem Säurechloridverfahren oder nach dem bereits erwähnten Verfahren der Direktkondensation aus den gleichen Edukten wie oben beschrieben. Bedingt durch einen Überschuß an Fettsäure bei der Kondensation und eine nicht vollständige Umsetzung, enthält ein solches Acyloxialkansulfonat noch mehr oder weniger große Restanteile freier Fettsäuren.

Die Zumischung des zweiten Acyloxialkansulfonats erfolgt vor, während oder nach der Herstellung des ersten Acyloxialkansulfonats. Vorzugsweise geht man so vor, daß man zunächst ein Acyloxialkansulfonat herstellt und zu der noch heißen Reaktionsmischung das zweite Acyloxialkansulfonat entweder in fester oder in flüssiger Form mittels geeigneter Vorrichtungen zumischt, beispielsweise in einem Rührkessel oder mit Hilfe einer Misch-, Rühr-, Homogenisier- oder Hohlraumübertragungsvorrichtung, z.B. in einem Cavitron-Gerät. Es ist auch möglich, das Mischen getrennt vom Herstellungsprozess für das erste Acyloxialkansulfonate durchzuführen, indem man ein oder mehrere fertige Acyloxialkansulfonate aufschmilzt und zu der Schmelze das oder die zweiten Acyloxialkansulfonate in flüssiger oder fester Form zugibt und die Mischung anschließend homogenisiert.

Die auf die beschriebene Art und Weise hergestellten Tensidmischungen, die als Hauptkomponente Acyloxialkansulfonate mit gemischten Kationen und daneben Restmengen an freien Fettsäuren und gegebenenfalls Konsistenzregler enthalten, zeichnen sich gegenüber ähnlichen Produkten nach dem Stand der Technik (US 3,029,264) dadurch aus, daß bei gleichem Gehalt an Acyloxialkansulfonat die Temperaturgrenze, bei der das Tensidgemisch noch rührbar ist, deutlich niedriger ist. Diese Absenkung der Rührbarkeitsgrenze bei den erfindungsgemäßen Tensidmischungen hat zur Folge, daß hier der Gehalt an Acyloxialkansulfonat höher sein kann als bei den Mischungen des genannten Standes der Technik. Eine weitere Absenkung der Rührbarkeitsgrenze kann durch Zugabe von Konsistenzreglern erfolgen. Darüberhinaus ist bei den erfindungsgemäßen Tensidmischungen der Restgehalt an freiem Isethionat deutlich niedriger.

### Beispiel 1

In einem 2 l Planschliffbecher mit Rührer, absteigender Destillationsbrücke, Innenthermometer und Stickstoffeinleitung wurden 236 g Cocosfettsäure, 255 g wäßrige Natriumisethionat-Lösung (58 %) und 0,71 g Zinkoxid vorgelegt. Der Ansatz wurde auf 210°C erwärmt und das bei der Direktkondensation gebildete Wasser abdestilliert. Bei einem Gehalt an waschaktiver Substanz (WAS) von 78 % (Epton-Titration) wurden bei einer Temperatur von 178°C 56 g Ammoniumcocoylisethionat (84 %ige Ware, die zusätzlich Cocosfettsäure enthält) zugesetzt, das Gemisch 0,5 h bei etwa 170°C gerührt und anschließend langsam abgekühlt. Das so erhaltene Natrium-/Ammoniumcocoylisethionat-Gemisch war noch bei einer Temperatur von 105°C rührbar und enthielt 82 % waschaktive Substanz (Epton-Titration) und 4,3 % freies Hydroxiethansulfonat. Wurden zusätzlich 14,2 g Stearinsäure zugesetzt, war das Gemisch bei einem WAS-Gehalt von 74 % und einem Gehalt von 3,7 % Hydroxiethansulfonat noch bei einer Temperatur von 95°C rührbar.

Die in den folgenden Beispielen aufgeführten Gemische wurden hergestellt durch Aufschmelzen und Mischen der jeweiligen Acyloxyalkansulfonate. Beim Abkühlen dieser Mischungen wurden die in den jeweiligen Tabellen angegebenen Sinterpunkte und Schmelzbeginn gemessen. Der Gehalt an Acyloxialkansulfonat in diesen Mischungen beträgt mehr als 95 Gew.-%

### Beispiel 2

| K/NH₄-lauroylisethionat | | Sinterpunkt [°C] | Schmelzbeginn [°C] |
|---|---|---|---|
| K-Salz [%] | NH₄-Salz [%] | | |
| 0 | 100 | 150 | 160 |
| 25 | 75 | 140 | 150 |
| 50 | 50 | 150 | 162 |
| 75 | 25 | 168 | 178 |
| 85 | 15 | 170 | 190 |
| 90 | 10 | 179 | 190 |
| 95 | 5 | 192 | 207 |
| 100 | 0 | 227 | 235 |

### Beispiel 3

| Na/NH₄-lauroylisethionat | | Sinterpunkt [°C] | Schmelzbeginn [°C] |
|---|---|---|---|
| Na-Salz [%] | NH₄-Salz [%] | | |
| 0 | 100 | 150 | 160 |
| 25 | 75 | 150 | 162 |
| 30 | 70 | 152 | 165 |
| 50 | 50 | 157 | 173 |
| 75 | 25 | 161 | 181 |
| 85 | 15 | 185 | 195 |
| 90 | 10 | 195 | 202 |
| 95 | 5 | 198 | 210 |
| 100 | 0 | 220 | 230 |

### Beispiel 4

| Na/NH(Et₃)-lauroylisethionat | | Sinterpunkt [°C] | Schmelzbeginn [°C] |
|---|---|---|---|
| Na-Salz [%] | NHEt₃-Salz [%] | | |
| 0 | 100 | flüssig | |
| 25 | 75 | noch gießbar | |
| 50 | 50 | pastös | |
| 75 | 25 | wachsartig | |
| 85 | 15 | 160 | 191 |
| 90 | 10 | 173 | 200 |
| 95 | 5 | 185 | 205 |
| 100 | 0 | 220 | 230 |

### Beispiel 5

| K/NH(Et₃)-lauroylisethionat | | Sinterpunkt [°C] | Schmelzbeginn [°C] |
|---|---|---|---|
| K-Salz [%] | NHEt₃-Salz [%] | | |
| 0 | 100 | flüssig | |
| 25 | 75 | noch gießbar | |
| 50 | 50 | pastös | |
| 75 | 25 | wachsartig | |
| 85 | 15 | 178 | 206 |
| 90 | 10 | 190 | 211 |
| 95 | 5 | 197 | 217 |
| 100 | 0 | 227 | 235 |

## Patentansprüche

1. Tensidmischungen mit einem Gehalt an Acyloxialkansulfonaten, hergestellt durch Umsetzung einer oder mehrerer Fettsäuren mit mindestens einem Ammonium-, Alkali- und/oder Erdalkali-hydroxyalkansulfonat unter Bildung eines ersten Acyloxialkansulfonats, wobei vor, während oder nach der Herstellung dieses ersten Acyloxialkansulfonats mindestens ein zweites Ammonium-, Alkali- und/oder Erdalkali-acyloxialkansulfonat zugesetzt wird, wobei die Kationen in dem Hydroxyalkansulfonat und in dem zweiten Acyloxialkansulfonat so gewählt sind, daß die Gesamtmenge an Acyloxialkansulfonat an der Tensidmischung als gemischtes Ammoniumkation sowie Alkali- und/oder Erdalkalikation vorliegt.

2. Tensidmischungen nach Anspruch 1, dadurch gekennzeichnet, daß das Acyloxialkansulfonat ein Acylisethionat ist.

3. Tensidmischungen nach Anspruch 1, dadurch gekennzeichnet, daß das Hydroxialkansulfonat ein Ammonium-, Natrium- oder Kaliumhydroxialkansulfonat ist.

4. Tensidmischungen nach Anspruch 1, dadurch gekennzeichnet, daß das Ammoniumkation des Hydroxialkansulfonats und des Acyloxialkansulfonats der Formel
R¹R²R³R⁴N⊕
entspricht, worin R¹, R², R³ und R⁴ Wasserstoff, C₁-C₄-Alkyl oder C₁-C₄-Hydroxyalkyl bedeuten.

5. Tensidmischung nach Anspruch 1, hergestellt durch Umsetzung von Cocosfettsäure.

6. Tensidmischungen nach Anspruch 1, dadurch gekennzeichnet, daß die Gesamtmenge an Acyloxialkansulfonat als gemischtes Natrium- und Ammoniumsalz vorliegt mit einem molaren Verhältnis von Na zu NH₄ von 98:2 bis 5:95.
